# EUROPEAN PATENT SPECIFICATION

(11) **EP 2 907 530 B1**
(45) Date of publication and mention of the grant of the patent: **02.01.2019**
(21) Application number: 15154928.4
(22) Date of filing: 12.02.2015
(51) Int. Cl.: A61L 27/26, A61L 27/20, A61L 27/16, A61L 27/54, C08L 5/08, C08L 29/04, C08K 3/16, A61L 27/52, C08L 5/04

(54) **KIT TO CREATE GELLED STRUCTURES INSIDE THE HUMAN OR ANIMAL BODY FOR MEDICAL PURPOSES**
AUSRÜSTUNG ZUR HERSTELLUNG GELIERTER STRUKTUREN INNERHALB MENSCHLICHER ODER TIERISCHER KÖRPER FÜR MEDIZINISCHE ZWECKE
KIT POUR LA FORMATION DE STRUCTURES GELIFIÉES DANS LE CORPS HUMAIN OU ANIMAL À DES FINS MÉDICALES

(30) Priority: 12.02.2014 IT RM20140061
(43) Date of publication of application: 19.08.2015
(73) Proprietor: Niob, Sagl, 6900 Lugano (CH)
(72) Inventor: Matricardi, Pietro, 00139 Roma (IT); Cencetti, Claudia, 00142 Roma (IT)
(74) Representative: Bosman, Cesare

(56) References cited:
- EP-A1- 1 262 201
- WO-A2-2006/125082
- HUA S ET AL: "pH-sensitive sodium alginate/poly(vinyl alcohol) hydrogel beads prepared by combined Ca<2+> crosslinking and freeze-thawing cycles for controlled release of diclofenac sodium", INTERNATIONAL JOURNAL OF BIOLOGICAL MACROMOLECULES, ELSEVIER BV, NL, vol. 46, no. 5, 16 March 2010 (2010-03-16) , pages 517-523, XP027033412, ISSN: 0141-8130 [retrieved on 2010-03-16]

## Description

The present invention concerns a kit to create gelled structures inside the human or animal body for medical purposes.

The sterile gelled structures created with the kit of the present invention can be particularly advantageously applied in the field of dentistry, in cosmetic surgery or in filler and reconstruction surgery.

In the medical field it is necessary to be able to create a biocompatible structure inside the human or animal body in order to meet the needs of filling of cavities to obtain cell regeneration, to obtain an inert support or for the localised carrying of drugs.

The creation of said structure must firstly ensure that it is formed gradually and directly in situ. In fact, insertion in the body of a preformed structure would be extremely inconvenient, and also ineffective. The gradual formation of these structures gives the operator the time necessary to arrange the material in the area of interest with the certainty that, in the formation phase, said structures will occupy all the spaces (interstices) available.

Furthermore, said structures must have characteristics of adhesion such as to guarantee an effective durability in the chosen area.

It must also be possible to "load" said gelled structures either with a pharmacologically active ingredient which can then be gradually released, or with a biomaterial necessary to support the medical and/or surgical action.

Lastly, said gelled structures must be able to perform an auxiliary function in medical practice, as a simple system of temporary support to sustain tissues or immobilize devices.

"INTERNATIONAL JOURNAL OF BIOLOGICAL MACROMOLECULES, ELSEVIER BV, NL, vol. 46, no. 5 16 March 2010, pages 517-523, XP027033412" discloses the preparation of an hydrogel made from the cross linking reaction between PVA and alginate in the presence of calcium chloride. The reaction leads to the formation of sodium alginate/polyvinylalcohol hydrogel beads.
EP 1 262 201 A1 discloses a biodegradable hydrogel material comprising PVA and gellan gum for medical applications.
WO 2006/125082 A2 discloses a kit for providing a hydrogel composition, said kit comprising a first container of pre-gelled or pre-solidified hydrogel particles, wherein the hydrogel particles are loaded or embedded with at least one gallant, a second container containing a hydrogel solution and a delivery device such as a syringe with a needle.

The need was therefore felt for a solution for the creation of a gelled structure that met the above needs.

The subject of the present invention is a kit to create gelled structures inside the human or animal body for medical purposes, the essential characteristics of which are contained in claim 1, and the preferred and/or auxiliary characteristics of which are contained in claims 2-4.

Below embodiment examples are provided for purely illustrative nonlimiting purposes with the help of the figures of the accompanying drawing in which:
Figure 1 is a graph in which the viscosity values are reported as a function of the flow speed of the two solutions A and B, prepared according to example 1;
Figure 2 is a graph which contains a mechanical spectrum of the gelled structure obtained from contact of the two solutions A and B prepared according to example 1, the viscosity of which is shown in figure 1.

The mean molecular weight values of the polymer species reported in the present description and in the claims have been calculated with the method of the international standard ASTM D4001-13.

The viscosity values reported below were identified according to the method of DIN 53019-1

### - PREPARATION OF THE TWO SOLUTIONS A AND B -

### EXAMPLE 1

### - Preparation of solution A -

10 grams of polyvinyl alcohol (PVA, molecular weight approximately 145,000), as biocompatible polymeric agent with viscosizing mucoadhesive action, were suspended in 100 ml of bidistilled H₂O filtered using a filter with dimensions of 0.2 pm. The suspension was stirred (magnetically or mechanically), and heated to 90°C, until complete solubilization of the polymer. 2 grams of calcium chloride, CaCl₂ (or 2.65 grams of calcium chloride dihydrate, CaCl₂• 2H₂O) were added to the solution of PVA, and the resulting solution was left under hot stirring until complete solubilization.

### - Preparation of solution B -

2 grams of gellan (molecular weight approximately 10⁶), as biocompatible polymer with ionotropic gelation, were suspended in 100 ml of bidistilled H₂O filtered using a filter with dimensions of 0.2 µm. The suspension was stirred (magnetically or mechanically) and heated to 90°C until complete solubilization of the polymer.

### - Filling the syringes A -

A syringe made of glass (or other material adapted to withstand treatment in autoclave), provided with Luer-lock, was filled with 1 ml of solution A, still hot.

### - Filling the syringes B -

A syringe made of glass (or other material adapted to withstand treatment in autoclave), provided with Luer-lock, was filled with 1 ml of solution B, still hot.

### - Sterilization -

The syringes A and B were sterilized in an autoclave, at 121°C for 20 minutes.

### EXAMPLE 2

### - Preparation of solution A -

The preparation described for example 1 was repeated, the sole exception being that together with the CaCl₂, also 20 mg of Brilliant Blue FCF colourant (E133) were added to the PVA solution.

### - Preparation of solution B -

The preparation described for example 1 was repeated, the only exception being that, once complete solubilization of the gellan had been obtained, 20 mg of Orange Yellow S (E110) were added to the resulting solution. The solution thus obtained was left under hot stirring until complete solubilization.

### - Filling the syringes A and B and their sterilization -

The operations described in example 1 were repeated.

### EXAMPLE 3

### - Preparation of solution A -

The preparation described in example 1 was repeated, the exception being that 5 grams of polyvinyl alcohol were used (PVA, molecular weight approximately 195,000) and that 1 gram of calcium chloride, CaCl₂ (or 1.325 grams of calcium chloride dihydrate, CaCl₂•2H₂O) and 1 gram of sodium chloride, NaCl were added to the PVA solution.

### - Preparation of solution B -

The preparation described in example 1 was repeated.

### - Filling the syringes A and B and their sterilization -

The operations described in example 1 were repeated.

### EXAMPLE 4

### - Preparation of solution A -

The preparation described in example 1 was repeated, the exception being that as biocompatible polymeric agent with viscosizing and mucoadhesive action, 5 grams of hydroxyethylcellulose (HEC, molecular weight approximately 90,000) were used.

### - Preparation of solution B -

The preparation described in example 1 was repeated, the exception being that 1.5 grams of gellan were used.

### - Filling the syringes A and B and their sterilization -

The operations described in example 1 were repeated.

### EXAMPLE 5

### - Preparation of solution A -

The preparation described in example 1 was repeated, the exception being that 15 grams of polyvinyl alcohol (PVA, molecular weight approx. 100,000) were used and that instead of CaCl₂, 3 grams of sodium chloride NaCl were used.

### - Preparation of solution B -

The preparation described in example 1 was repeated, the exception being that as biocompatible polymer with ionotropic gelation, 3 grams of gellan functionalized with Lysine (molecular weight approximately 10⁶) were used.

### - Filling the syringes A and B and their sterilization -

The operations described in example 1 were repeated.

### EXAMPLE 6

### - Preparation of solution A -

The preparation described in example 1 was repeated, the exception being that 10 grams of polyvinylpyrrolidone (PVP, molecular weight approximately 400,000) were used and 1 gram of calcium chloride, CaCl₂ (or 1.325 grams of calcium chloride dihydrate, CaCl₂•2H₂O) and 1 gram of sodium chloride NaCl were added to the PVP solution.

### - Preparation of solution B -

The preparation described in example 1 was repeated, the exception being that as biocompatible polymer with ionotropic gelation, 3 grams of alginate (molecular weight approximately 3x10⁵) were used.

### - Filling the syringes A and B and their sterilization -

The operations described in example 1 were repeated.

### EXAMPLE 7

### - Preparation of solution A -

The preparation described in example 1 was repeated, the exception being that 5 grams of polyvinyl alcohol (PVA, molecular weight approximately 195,000) were used, and that 1 gram of calcium chloride, CaCl₂ (or 1.325 grams of calcium chloride dihydrate, CaCl₂• 2H₂O), 1 gram of sodium chloride NaCl and 50 milligrams of Acesulfame K (E950) were added to the PVA solution.

### - Preparation of solution B -

The preparation described in example 1 was repeated.

### - Filling the syringes A and B and their sterilization -

The operations described in example 1 were repeated.

In the above examples it was verified that the solutions A and B were able to withstand the sterilization conditions adopted without modification of the chemical-physical characteristics necessary for production of the gelled structure.

Unlike the examples described above, the solution A and B can be inserted in respective tubular injection vials or vials instead of in syringes.

### - PREPARATION OF THE GELLED STRUCTURE -

The gelled structure is obtained by simple contact between the two solutions A and B.

In a typical preparation, the solution A is extruded with a needle until partial filling of a cavity, or until a surface is covered. Immediately after, the solution B is extruded on the solution A: the two solutions in contact gradually become a gelled structure by means of ionotropic gelation, by interaction between the polymer contained in the solution B and the mono- and/or divalent salt contained in the solution A. The gelled structure forms gradually thanks to the disturbing action exerted by the polymer of the solution A on the interaction between the polymer contained in the solution B and the salt contained in the solution A during the ionotropic gelation and its elasticity increases over time, until it becomes stable after approximately 5 minutes.

In another preparation, the two solutions are alternatively extruded dropwise; one drop of solution A is extruded with a needle in a cavity, or on a surface; immediately after, a drop of solution B is extruded on said solution A, also in this case leading to the formation of a gelled structure. The operation is continued alternating drops of solution A and solution B until complete filling of the cavity or complete covering of the surface.

In another preparation, the solutions A and B can be inverted, extruding the solution B on the solution A.

### - TESTS ON CHARACTERIZATION OF THE SOLUTIONS AND OF THE RESULTING GELLED STRUCTURE -

Two solutions A and B were prepared following the procedures given in example 1.

The two solutions were characterised by extrusion measurements through 18 and 20 gauge needles, by means of a Texture Analyzer TA-XT2i dynamometer. Table I shows the extrusion measurement values expressed in Newton.

**TABLE I**

| | Ago 18 G | Ago 20 G |
|---|---|---|
| Solution A (Example 1) | 7.56 ± 0.08 N | 27.41 ± 0.51 N |
| Solution B (Example 1) | 1.69 ± 0.28 N | 1.84 ± 0.22 N |

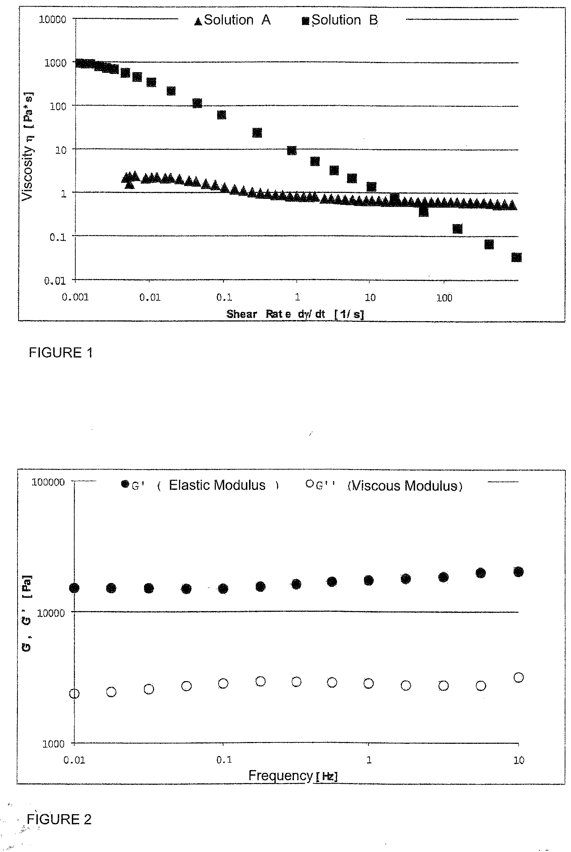

The solutions A and B of example 1 were further characterised by viscosity measurements by means of rheological analysis with a rotational rheometer (Haake RheoStress 300, cone/plate geometry Haake CP60/1° Ti - diameter = 60 mm - cone = 1°, flow speed = 0.001-1000 s⁻¹, T = 25°C) (Figure 1) .

The two solutions A and B were combined to form the gelled structure: 1 ml of solution A was poured onto the knurled plate of a geometry of the rheometer until it completely covered the surface; subsequently, 1 ml of solution B was poured onto the surface of the solution A. After 5 minutes, once the gelled structure had completely formed, the mechanical spectrum of the latter was measured (Haake RheoStress 300, flat geometry/knurled plate Haake PP35 Ti - diameter = 35 mm, f = 0.01-10 Hz, γ = 0.01, T = 25°C) (Figure 2).

The diagram of figure 1 clearly shows that solution A is a very viscous solution (approximately 1000 times distilled water at 25°C) but still liquid; this has the great advantage of ensuring that solution A does not rapidly run off the site of application but is simultaneously able to occupy the entire volume to be filled, occupying the recesses and following the bumps of the area to be filled, leaving the operator time to perform the various operations necessary.

Figure 1 furthermore shows that solution B is also a very viscous fluid, but with pseudoplastic behaviour such that it can be easily extruded by a syringe and therefore spread on the solution A without encountering application problems.

Lastly, figure 2 highlights the rheological-mechanical characteristics of the gelled structure obtained from interaction of the two solutions (parallel elastic and viscous modules with G' greater than G") with good mechanical properties.

The properties described above of the solutions A and B can also be exploited to prepare the gelled structure in appropriate containers outside the human body and, for example, be mixed with other substances or biomaterials and then subsequently introduced on the application site according to the specific medical technique, before complete gelation.

It has been verified that to produce a gelled structure as described above, certain viscosity characteristics of the two solutions and certain concentrations of the polymers and of the mono or plurivalent ion must be observed.

In fact, the conditions reported in the first claim guarantee the formation of a gelled structure characterised by a prolonged gelation time (the mixing of the two solutions results in adequate gelation in 1 minute and complete gelation in 5 minutes) so as to allow the doctor to mix the two solutions in vitro, add (when necessary) inert or pharmacologically active substances, and position the mixture in situ before the gelation is complete. Alternatively, the two solutions can also be applied directly in situ having previously added the inert or pharmacologically active substance to any one of the two solutions. Furthermore, the gelled structure obtained with the kit of the present invention is like a weak gel, which is non-rigid, spreadable and mucoadhesive.

In this specific case, the inventors have surprisingly found that the polymeric agent with viscosizing and mucoadhesive action, if used observing the concentration interval indicated in the claims, is able, without cross-linking, to guarantee a functional interference in the gelation between the ion and the polymer of the other water solution. In this way the gelation time is increased, the final gelled structure is weakened and, at the same time, the resulting gelled structure is given the required mucoadhesive characteristics.

From the above description it is immediately clear that the kit subject of the present invention is able to produce a uniform gelled structure which, thanks to the characteristics of viscosity and mucoadhesion of the solution A, is able to form also in areas difficult to reach with the normal techniques and position itself in a stable manner.

It has been experimentally proved that the uniformity of the gelled structure favours cell interaction and, therefore, tissue formation.

Furthermore, one of the two solutions, A or B, can be "loaded" with a pharmacologically active ingredient, so that the resulting gelled structure can release this pharmacological ingredient in a localised manner.

In said regard, the kit can preferably contain a third syringe C filled with a third water solution or suspension C containing a pharmacologically active ingredient or a biomaterial and connection means for connecting the syringe A or B to the syringe C in order to mix the solution A or B with the solution or suspension C.

Alternatively, the kit can preferably contain a third empty container C (syringe or tubular injection vial or vial), to be filled as required with a solution or suspension of a pharmacologically active ingredient or a biomaterial, and connection means for connecting the syringe A or B to the container C, in order to mix the solution A or B with the solution or suspension C.

Example 2 has a further advantage which enables the operator to visually verify that gelation has taken place. In fact, due to the presence of a colourant in each of the two solutions A and B, the formation of a uniform colouring resulting from the combination of the two colours constitutes visual evidence that the gelation has taken place.

Lastly, the present invention can be advantageously applied in all sectors of medicine where it can be useful to have a gelled structure produced in situ and which can allow localised release of a pharmacologically active ingredient, a support for the deposition of biomaterials, including any contrast agents for analysis, and/or cell re-formation. In particular, the present invention can be applied in dentistry, in cosmetic surgery and in reconstruction surgery.

## Claims

1. A kit to create sterile gelled structures inside the human or animal body for medical purposes, said kit being **characterised in that** it comprises:
- a first container containing a first water solution having at ambient temperature a viscosity in the range from 0.1 to 10 Pa·s and comprising at least 8 to 12% by weight/volume of a biocompatible polymeric agent with viscosizing and mucoadhesive action and 1 to 3% by weight/volume of a mono or plurivalent ion;
- a second container containing a second water solution having at ambient temperature a viscosity in the range from 0.1 to 100 Pa·s at 1 s⁻¹ and comprising at least 1 to 3% by weight/volume of gellan polymer adapted to gel with said ion of said first water solution by ionotropic gelation; said biocompatible polymeric agent with viscosizing and/or mucoadhesive action being chosen from polyvinyl alcohol, polyvinylpryrrolidone and hydroxyethylcellulose;
said first and said second container being adapted to be subject to a sterilisation operation; said biocompatible polymeric agent with viscosizing and/or mucoadhesive action having a mean molecular weight in the range from 5 x 10⁴ to 4 x 10⁵ and said gellan polymer of said second water solution having a mean molecular weight in the range from 1x10⁵ to 2.5x10⁶.

2. Kit according to claim 1, **characterised in that** it comprises a third container adapted to be filled with a third water solution or suspension containing a pharmacologically active substance or a biomaterial and connection means for connecting said first or second container to said third container to mix said first or second solution and said third water solution or suspension.

3. Kit according to one of the preceding claims, **characterised in that** said first and/or said second water solution comprise a respective colouring agent and/or a radio-opaque agent.

4. Kit according to one of the preceding claims, **characterised in that** said first and/or said second water solution comprise a taste-modifier agent.

## Patentansprüche

1. Ein Kit zur Herstellung steriler gelierter Strukturen innerhalb menschlicher oder tierischer Körper für medizinische Zwecke, wobei das Kit **dadurch gekennzeichnet ist, dass** es umfasst:
- einen ersten Behälter, der eine erste wässrige Lösung mit einer Viskosität bei Umgebungstemperatur im Bereich von 0,1 bis 10 Pa·s enthält und mindestens 8 bis 12 % w/v eines biokompatiblen polymeren Mittels mit viskositätseinstellender und mukoadhäsiver Wirkung und 1 bis 3 % w/v eines ein- oder mehrwertigen Ions umfasst;
- ein zweiter Behälter, der eine zweite wässrige Lösung mit einer Viskosität bei Umgebungstemperatur im Bereich von 0,1 bis 100 Pa·s bei 1 s⁻¹ enthält und mindestens 1 bis 3 % w/v Gellanpolymer, das geeignet ist, mit dem Ion der ersten Wasserlösung durch ionotrope Gelierung zu gelieren, umfasst; wobei das biokompatible polymere Mittel mit viskositätseinstellender und/oder mukoadhäsiver Wirkung aus Polyvinylalkohol, Polyvinylpyrrolidon und Hydroxyethylcellulose ausgewählt ist;
wobei der erste und der zweite Behälter dafür geeignet sind, einer Sterilisierungsbehandlung unterzogen zu werden; wobei das biokompatible polymere Mittel mit viskositätseinstellender und/oder mukoadhäsiver Wirkung ein mittleres Molekulargewicht im Bereich von 5 × 10⁴ bis 4 × 10⁵ aufweist und wobei das Gellanpolymer der zweiten wässrigen Lösung ein mittleres Molekulargewicht im Bereich von 1 × 10⁵ bis 2,5 × 10⁶ aufweist.

2. Das Kit gemäß Anspruch 1, **dadurch gekennzeichnet, dass** es einen dritten Behälter, der dazu geeignet ist, mit einer dritten wässrigen Lösung oder Suspension gefüllt zu werden, die eine pharmakologisch wirksame Substanz oder ein Biomaterial enthält, und Verbindungsmittel zum Verbinden des ersten oder zweiten Behälters mit dem dritten Behälter, um die erste oder zweite Lösung und die dritte wässrige Lösung oder Suspension zu mischen, umfasst.

3. Das Kit gemäß einem der vorstehenden Ansprüche, **dadurch gekennzeichnet, dass** die erste und/oder zweite wässrige Lösung ein entsprechendes Färbemittel und/oder ein strahlenundurchlässiges Mittel umfasst.

4. Das Kit gemäß einem der vorstehenden Ansprüche, **dadurch gekennzeichnet, dass** die erste und/oder zweite wässrige Lösung ein den Geschmack veränderndes Mittel umfasst.

## Revendications

1. Kit pour créer des structures gélifiées stériles à l'intérieur du corps humain ou animal à des fins médicales, ledit kit étant **caractérisé en ce qu'**il comprend :
- un premier récipient contenant une première solution aqueuse ayant, à température ambiante, une viscosité située dans la plage allant de 0,1 à 10 Pa.s et comprenant au moins 8 à 12 % en poids/volume d'un agent polymère biocompatible ayant une action viscosifiante et mucoadhésive et 1 à 3 % en poids/volume d'un ion monovalent ou polyvalent ;
- un deuxième récipient contenant une deuxième solution aqueuse ayant, à température ambiante, une viscosité située dans la plage allant de 0,1 à 100 Pa.s à 1s⁻¹ et comprenant au moins 1 à 3 % en poids/volume de polymère de gellane adapté pour gélifier avec ledit ion de ladite première solution aqueuse par gélification ionotrope ; ledit agent polymère biocompatible ayant une action viscosifiante et/ou mucoadhésive étant choisi parmi le poly(alcool vinylique), la polyvinylpyrrolidone et l'hydroxyéthylcellulose ;
lesdits premier et deuxième récipients étant adaptés pour faire l'objet d'une opération de stérilisation ; ledit agent polymère biocompatible ayant une action viscosifiante et/ou mucoadhésive ayant une masse moléculaire moyenne située dans la plage allant de 5 x 10⁴ à 4 x 10⁵, et ledit polymère de gellane de ladite deuxième solution aqueuse ayant une masse moléculaire moyenne située dans la plage allant de 1 x 10⁵ à 2,5 x 10⁶.

2. Kit selon la revendication 1, **caractérisé en ce qu'**il comprend un troisième récipient adapté pour être rempli d'une troisième solution ou suspension aqueuse contenant une substance présentant une activité pharmacologiquement active ou un biomatériau et des moyens de connexion pour connecter ledit premier ou deuxième récipient audit troisième récipient pour que ladite première ou deuxième solution soit mélangée avec ladite troisième solution ou suspension aqueuse.

3. Kit selon l'une des revendications précédentes, **caractérisé en ce que** lesdites première et/ou deuxième solutions aqueuses comprennent un agent colorant respectif et/ou un agent radio-opaque.

4. Kit selon l'une des revendications précédentes, **caractérisé en ce que** lesdites première et/ou deuxième solutions aqueuses comprennent un agent modificateur de goût.
